# EUROPÄISCHE PATENTSCHRIFT

(11) **EP 2 337 516 B1**
(45) Veröffentlichungstag und Bekanntmachung des Hinweises auf die Patenterteilung: **06.01.2016**
(21) Anmeldenummer: 09777840.1
(22) Anmeldetag: 12.08.2009
(51) Int. Cl.: A61B 18/12, A61B 18/16, A61B 18/14

(54) **ELEKTROCHIRURGISCHER HF-GENERATOR**
ELECTROSURGICAL HF GENERATOR
GÉNÉRATEUR ÉLECTROCHIRURGICAL HAUTE FRÉQUENCE

(30) Priorität: 01.10.2008 DE 102008050032; 03.11.2008 DE 102008054351
(43) Veröffentlichungstag der Anmeldung: 29.06.2011
(73) Patentinhaber: ERBE Elektromedizin GmbH, 72072 Tübingen (DE)
(72) Erfinder: BELLER, Jürgen, 72810 Gomaringen (DE); KLEIN, Ralf, 72108 Rottenburg (DE)
(74) Vertreter: Bohnenberger, Johannes
(86) Internationale Anmeldenummer: PCT/EP2009/005856
(87) Internationale Veröffentlichungsnummer: WO 2010/037446

(56) Entgegenhaltungen:
- EP-A1- 0 694 291
- EP-A1- 1 902 684
- WO-A1-2004/045436
- WO-A1-2007/022864
- WO-A2-2005/110263
- DE-A1- 10 351 818
- US-A- 4 754 757
- US-A- 5 766 165
- US-A1- 2004 097 916
- US-A1- 2005 101 947
- US-A1- 2008 058 792

## Beschreibung

Die Erfindung betrifft einen elektrochirurgischen HF-Generator nach dem Oberbegriff des Patentanspruches 1.

Elektrochirurgische HF-Generatoren werden zur Behandlung, insbesondere zum Schneiden oder zum Koagulieren von biologischem Gewebe eingesetzt. Dazu ist eine Generatorschaltung zum Erzeugen eines hochfrequenten HF-Stroms vorgesehen, der zwischen einer Aktivelektrode und mindestens einer Neutralelektrode über einen ersten Strompfad durch das Gewebe fließt. Entsprechend umfasst die Generatorschaltung einen Aktivausgang und einen Neutralausgang.

Bei der HF-Chirurgie wird zwischen so genannter monopolarer Anwendung und so genannter bipolarer Anwendung unterschieden. Bei der monopolaren Anwendung ist die Aktivelektrode als isoliertes Handstück ausgeführt, die z. B. eine Elektrodenspitze aufweist, welche von einem Operateur zu den zu behandelnden Gewebebereichen geführt wird. Dort fließt ein HF-Strom durch das Gewebe zu einer das Gewebe großflächig kontaktierenden Neutralelektrode, die z. B. am Oberschenkel eines Patienten angebracht ist. Solche Neutralelektroden haben üblicherweise eine Kontaktfläche zwischen etwa 1 bis etwa 3 dm². Die monopolare Anwendung hat den Vorteil eines kompakten Handstückes, dessen Elektrodenspitze auch an nur schwer zu erreichenden Gewebestellen angesetzt werden kann. Nachteilig ist, dass der HF-strom auch durch nicht zu behandelndes Gewebe zu der Neutralelektrode fließt. Insbesondere dann, wenn der Querschnitt des Gewebes entlang des entsprechenden Strompfades gering ist, kann es zu einer Überwärmung und damit zu einer Schädigung von nicht zu behandelnden Bereichen des Gewebes kommen. Dem kann durch die bipolaren Instrumente begegnet werden. Bei bipolaren Instrumenten fließt der HF-Strom zwischen zwei nahe beieinander liegenden Elektroden, z. B. einer bipolaren Koagulationszange, d.h. ausschließlich durch zwischen den Klemmflächen befindliches Gewebe. Schlecht zugängliche Gewebestellen können oftmals nur schlecht oder sogar gar nicht mit solchen Instrumenten erreicht und behandelt werden. US 5 766 165 wird als nächstliegender Stand der Technik angesehen und offenbart dem Oberbegriff von Anspruch 1.

Der Erfindung liegt die Aufgabe zugrunde, einen elektrochirurgischen HF-Generator bereitzustellen, der ohne Gefahr einer Schädigung von nicht zu behandelndem Gewebe eine Behandlung auch schwierig zu erreichender Gewebestellen ermöglicht.

Die Aufgabe wird durch einen HF-Generator nach dem Anspruch 1 gelöst. Vorteilhafte Ausführungsformen sind in den abhängigen Ansprüchen angegeben.

Der elektrochirurgische HF-Generator zur Behandlung, insbesondere zum Schneiden oder Koagulieren von biologischem Gewebe, erzeugt mittels einer Generatorschaltung einen hochfrequenten HF-Strom, der zwischen einer Aktivelektrode und mindestens einer Neutralelektrode über einen ersten Strompfad durch das Gewebe fließt. Der elektrochirurgische HF-Generator hat entsprechend einen Aktivausgang und einen Neutralausgang. Zusätzlich hat der HF-Generator mindestens eine zu der Neutralelektrode parallel geschaltete Hilfsneutralelektrode, so dass ein Teil des HF-Stroms über mindestens einen zweiten Strompfad gezielt durch das Gewebe fließt. Weil der gesamte HF-Strom auf mindestens zwei Strompfade verteilt wird, ist die Stromdichte in den Strompfaden entsprechend reduziert. In der Folge wird das Gewebe entlang der Strompfade, wenn überhaupt, dann nur gering erwärmt. Die Gefahr einer Gewebeschädigung durch Überhitzung ist entsprechend reduziert.

Vorzugsweise umfasst der elektrochirurgische HF-Generator eine Messschaltung, die das Verhältnis der über die Neutralelektrode und über die Hilfsneutralelektrode fließenden Ströme misst. Alternativ oder optional kann die Messschaltung den über die Neutralelektrode und/oder den über die Hilfsneutralelektrode fließenden HF-Strom messen. Die Messschaltung ermöglicht es, festzustellen, ob der HF-Strom so auf die beiden Strompfade aufgeteilt wird, dass unerwünschte Gewebeschädigungen vermieden werden. Besteht eine Gefahr einer unerwünschten Gewebeschädigung, kann diese angezeigt werden und/oder der HF-Generator selbsttätig abschalten. In den meisten Fällen kann durch eine Neupositionierung insbesondere der Hilfsneutralelektrode die Gefahr behoben werden.

Die Hilfsneutralelektrode umfasst mindestens eine Klemmfläche einer vorzugsweise selbsthaltenden Fasszange. Dies ist insbesondere dann vorteilhaft, wenn von dem Gewebe ein Teil abgetrennt werden soll. Dieser kann mit der Fasszange gegriffen werden, so dass der zusätzliche Strompfad von der Aktivelektrode durch den abzutrennenden Gewebeteil zu der Klemmfläche der Fasszange führt. Eine Schädigung von zu erhaltendem Gewebe durch den zusätzlichen Strompfad ist dadurch

ausgeschlossen. Sofern der abzutrennende Teil des Gewebes histologisch untersucht werden soll, darf der abzutrennende Teil des Gewebes auch nicht geschädigt werden. Dies kann durch eine Messschaltung, insbesondere durch eine der hierin beschriebenen Messschaltungen überwacht werden.

Bevorzugt sind beide Klemmflächen der Fasszange mit der Generatorschaltung verbunden sind. Wenn beide Klemmflächen der Fasszänge zu der Neutralelektrode parallel geschaltet sind, verdoppelt sich die Kontaktfläche der Hilfselektrode, woraus eine entsprechend geringere Belastung des durch die Klemmflächen kontaktierten Gewebebereichs resultiert.

Die Fasszange ist als bipolare Fasszange ausgebildet. Dann kann der elektrochirurgische HF-Generator eine Messschaltung zur Bestimmung des Widerstandes zwischen den beiden Klemmflächen der Fasszange umfassen. Mittels der Messschaltung kann erkannt werden, ob die Klemmflächen der Fasszange das Gewebe ausreichend kontaktieren.

Wenn der elektrochirurgische HF-Generator einen Wahlschalter zum Verbinden einer Klemmfläche der bipolaren Fasszange mit dem Aktivausgang und zum Verbinden der anderen Klemmfläche der bipolaren Fasszange mit dem Neutralausgang umfasst, dann kann die Betriebsart des HF-Generators durch Betätigen des Wahlschalters von monopolarer Anwendung auf bipolare Anwendung umgeschaltet werden, wodurch der Anwendungsbereich des HF-Generators entsprechend vergrößert wird. Bevorzugt hat der HF-Generator einen Wahlschalter zum Trennen der dann verbleibenden Elektroden, d.h. der Aktivelektrode und der Neutralelektrode, von der Generatorschaltung. Dies-dient der Unfallverhütung.

Vorzugsweise hat der elektrochirurgische HF-Generator einen Schalter zum Trennen der Hilfsneutralelektrode von der Generatorschaltung, weil nicht bei allen Operationssituationen eine Hilfsneutralelektrode am Gewebe angebracht werden kann.

Wenn der elektrochirurgische HF-Generator eine Messschaltung zum Bestimmen des elektrischen Widerstandes zwischen der Neutralelektrode und der Hilfsneutralelektrode umfasst, dann kann bevorzugt vor der eigentlichen Behandlung, d.h. bevor ein Operateur das zu behandelnde Gewebe schneidet oder koaguliert, überprüft werden, ob sowohl die Neutralelektrode als auch die Hilfsneutralelektrode korrekt am Gewebe angebracht sind, d.h. dieses ausreichend kontaktieren.

Anhand der Zeichnungen werden Ausführungsbeispiele der Erfindung schematisch vereinfacht und beschrieben. Es zeigen
- Fig. 1:: eine erste Ausführungsform eines elektrochirurgischen HF-Generators,
- Fig. 2:: eine zweite Ausführungsform eines elektrochirurgischen HF-Generators,
- Fig. 3:: einen weiteren elektrochirurgischen HF-Generator und
- Fig. 4:: einen elektrochirurgischen HF-Generator für monopolare und bipolare Anwendung.

In der nachfolgenden Beschreibung werden für gleiche und gleich wirkende Teile dieselben Bezugsziffern verwendet.

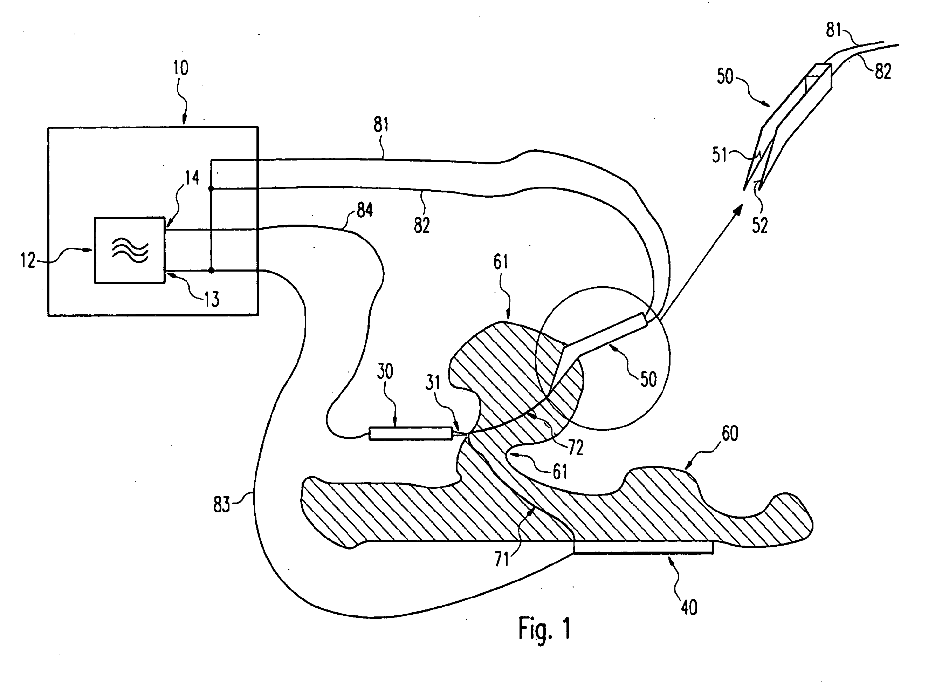

Der HF-Generator 10 in Fig. 1 umfasst eine Generatorschaltung 12 mit einem Neutralausgang 13 und einem Aktivausgang 14. Der Neutralausgang 13 ist über eine Leitung 83 mit einer ein Gewebe 60 großflächig kontaktierenden Neutralelektrode 40 verbunden. Zusätzlich ist der Neutralausgang 13 über Leitungen 81, 82 mit zwei als Hilfsneutralelektroden dienenden Klemmflächen 51, 52 einer bipolaren Fasszange 50 verbunden, welche einen abzutrennenden Teil 61 des Gewebes 60 kontaktiert. Der Aktivausgang 14 ist über eine Leitung 84 mit einer als Handstück ausgeführten Aktivelektrode 30 verbunden, die eine Elektrodenspitze 31 umfasst. Durch einen nicht dargestellten Schalter, der z.B. als Fußtaster oder als Handtaster an der Aktivelektrode 30 ausgeführt sein kann, kann ein Operateur den Stromkreis schließen, so dass ein HF-Strom zwischen dem Aktivausgang 14 und dem Neutralausgang 13 der Generatorschaltung 12 fließt. Dieser HF-Strom teilt sich auf zwei Strompfade 71, 72 durch das Gewebe 60 auf. Ein erster Strompfad 71 verläuft von der Elektrodenspitze 31 zu der Neutralelektrode 40. Ein zweiter Strompfad 72 verläuft von der Elektrodenspitze 31 zu den Hilfsneutralelektrode, d.h. hier zu den Klemmflächen 51, 52 der bipolaren Fasszange 50. Entsprechend ist die Stromdichte entlang des ersten Strompfades 71, welcher über einen Gewebebereich 62 mit verjüngter Querschnittsfläche führt, gegenüber monopolarer elektrochirurgischen Behandlung mit einem HF-Generator nach dem Stand der Technik, d.h. ohne die als Hilfsneutralelektrode fungierenden Klemmflächen 51, 52 der Fasszange 50 reduziert. Entsprechend werden die nicht zu behandelnden Gewebebereiche entlang des ersten Strompfades 71, insbesondere in dem Gewebebereich 62, entsprechend weniger erwärmt.

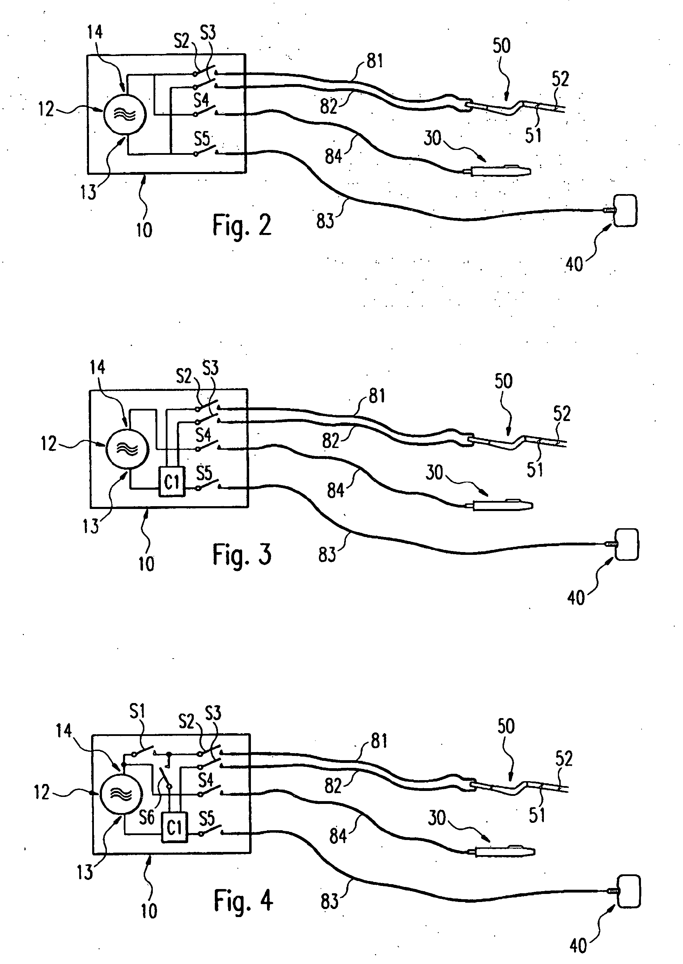

Der elektrochirurgische HF-Generator 10 gemäß Fig. 2 ist ähnlich wie der nach Fig. 1 aufgebaut, kann jedoch sowohl monopolar als auch bipolar betrieben werden. Zum monopolaren Betrieb werden Schalter S4 und S5 geschlossen (S2 und S3 in Offenstellung), dann ist eine Neutralelektrode 40 mit dem Neutralausgang 13 und eine Aktivelektrode 30 mit dem Aktivausgang 14 verbunden. Alternativ können Schalter S2 und S3 geschlossen werden (S4 und S5 in Offenstellung), dann ist eine Klemmfläche 52 einer bipolaren Fasszange 50 über eine Leitung 82 mit dem Neutralausgang 13 und die andere Klemmfläche 51 der bipolaren Fasszange 50 über eine Leitung 81 mit dem Aktivausgang 14 verbunden. Bei geschlossenen Schaltern S2, S3 kann der HF-Generator folglich mit bipolaren Instrumenten benutzt werden.

Der HF-Generator 10 gemäß Fig. 3 entspricht im Wesentlichen dem HF-Generator in Fig. 1, weshalb lediglich Unterschiede erläutert werden. Mittels der Schalter S2, S3 und S5 können die Neutralelektrode 40, bzw. die als Hilfsneutralelektroden dienenden Klemmflächen 51, 52 der bipolaren Fasszange 50 mit dem Neutralausgang 13 der Generatorschaltung 12 verbunden werden. Die entsprechenden Leitungen 81, 82, 83 von dem Neutralausgang 13 zu den Klemmflächen 51, 52 bzw. zu der Neutralelektrode 40 sind über eine Messschaltung C1 geführt. Die Messschaltung C1 bestimmt die Ströme durch die Klemmflächen 51, 52 und den Strom durch die Neutratelektrode 40, sowie das Verhältnis der Ströme. Ist einer der gemessenen Werte außerhalb eines entsprechenden vorher eingestellten Wertebereichs, dann wird dies dem Operateur signalisiert, z.B. durch eine nicht dargestellte Anzeige. Bevorzugt misst die Schaltung C1 vor der eigentlichen elektrochirurgischen Behandlung den Widerstand zwischen der Neutralelektrode 40 und den beiden Klemmflächen 51, 52 der Hilfsneutralelektröde. Liegt dieser Widerstand über einem zuvor festgesetzten Grenzwert, kann der Schalter S4 zwischen der Aktivelektrode 30 und dem Aktivausgang 14 in Offenstellung gesperrt und/oder dem Operateur über ein entsprechendes Warnsignal, z.B. eine LED signalisiert werden, dass er den Sitz der Neutralelektrode 40 und Hilfsneutralelektrode(n) überprüfen muss. Wenn bei einer Operation eine Hilfsneutralelektrode nicht angebracht werden kann, bleiben die Schalter S2 und S3 geöffnet.

Der elektrochirurgische HF-Generator 10 der Erfindung in Fig. 4 umfasst wie der elektrochirurgische HF-Generator in Fig. 3 die Generatorschaltung 12 mit einem Neutralausgang 13 und einem Aktivausgang 14, die über eine ganze Reihe von Schaltern S1 bis S6 mit einer als Handstück ausgebildeten Aktivelektrode 30, bzw. einer Neutralelektrode 40 und/oder den Klemmflächen 51, 52 einer bipolaren Fasszange 50 verbunden werden können. Die Schalter S1 bis S6 erlauben es die genannten Elektroden so mit dem Aktiv- bzw. Neutralausgang 13, 14 zu verbinden , so dass mit dem elektrochirurgischen Generator 10 sowohl monopolare elektrochirurgische Behandlungen (mit und ohne Hilfselektrode(n)), als auch bipolare elektrochirurgische Behandlungen durchführbar sind: Für eine herkömmliche monopolare elektrochirurgische Behandlung werden lediglich die Schalter S4 und S5 geschlossen. Dann ist die Aktivelektrode 30 mit dem Aktivausgang 14 und die Neutralelektrode 40 mit dem Neutralausgang 13 der Generatorschaltung 12 verbunden. Wenn zusätzlich die Schalter S2, S3 und S6 geschlossen werden, sind die Klemmflächen 51, 52 der bipolaren Fasszange 50 ebenfalls mit dem Neutralausgang 13 der Generatorschaltung 12 verbunden und dienen als Hilfsneutralelektrode. In dieser Betriebsart wird durch eine schon anhand von Fig. 3 beschriebene Messschaltung C1 der korrekte Sitz der Neutralelektrode 40 und der Hilfsneutralelektroden 51, 52 überwacht. Wenn die Schalter S4, S5 und S6 geöffnet und Schalter S1, S2 und S3 geschlossen sind, ist eine Klemmfläche 52 der bipolaren Fasszange 50 mit dem Neutralausgang 13 der Generatorschaltung 12 und die andere Klemmfläche 51 mit dem Aktivausgang 14 der Generatorschaltung 12 verbunden. Bei dieser Einstellung der Wahlschalter S1 bis S6 können mit dem bipolaren Instrument 50 wie gewohnt bipolare elektrochirurgische Behandlungen durchgeführt werden. Durch die Wahlschalter S1 bis S6 bietet der elektrochirurgische HF-Generator 10 nach Fig. 4 die Möglichkeit, monopolare elektrochirurgische Behandlungen mit der Aktivelektrode 30 und mit der Neutralelektrode 40 durchzuführen. Zusätzlich können Klemmflächen 51, 52 einer bipolaren Fasszange 50 als Hilfsneutralelektroden hinzugeschaltet werden. Alternativ ermöglicht der elektrochirurgische HF-Generator 10 auch eine bipolare elektrochirurgische Behandlung mittels des bipolaren Instruments 50. Die Schalter S1 bis S6 sind hier nur symbolisch dargestellt. Zur Vereinfachung der Bedienung, insbesondere zur Vermeidung von Fehlbedienungen werden die Schalter S1 bis S6 bevorzugt über ein von einem Operateur zu bedienenden Betriebsartenwahlement gesteuert.

### Bezugszeichenliste

- 10: elektrochirurgischer HF-Generator
- 12: Generatorschaltung
- 13: Neutralausgang
- 14: Aktivausgang
- 30: als Handstück ausgeführte Aktivelektrode
- 31: Elektrodenspitze der Aktivelektrode 30
- 40: Neutralelektrode
- 50: bipolare Fasszange
- 51, 52: Klemmflächen der bipolaren Fasszange 50,
- 60: biologisches Gewebe
- 61: abzutrennender Teil des biologischen Gewebes 60
- 62: Bereich des biologischen Gewebes 60 mit verjüngtem Querschnitt
- 71: Strompfad zwischen Aktiv-und Neutralelektrode
- 72: Strompfad zwischen Aktiv- Hilfsneutralelektrode(n)
- 81: Verbindungsleitung zur Hilfsneutralelektrode bzw. Klemmfläche 51
- 82: Verbindungsleitung zur Hilfsneutralelektrode bzw. Klemmfläche 52
- 83: Verbindungsleitung zur Neutralelektrode 40
- 84: Verbindungsleitung zur Aktivelektrode 30

## Patentansprüche

1. Elektrochirurgischer HF-Generator (10) zur Behandlung, insbesondere zum Schneiden oder zum Koagulieren, von biologischem Gewebe (60) mittels einem von einer Generatorschaltung (12) erzeugten hochfrequentem HF-Strom, der zwischen einer Aktivelektrode (30) und mindestens einer das Gewebe großflächig kontaktierenden Neutralelektrode (40) über einen ersten Strompfad durch das Gewebe (60) fließt, mit einem Aktivausgang (14) und einem Neutralausgang (13),
wobei der elektrochirurgische HF-Generator (10) mindestens eine zu der Neutralelektrode (40) parallel geschaltete Hilfsneutralelektrode (50, 51, 52) hat, die mindestens eine Klemmfläche (51, 52) einer Fasszange (50) umfasst, so dass mindestens ein Teil des HF-Stroms gezielt über einen zweiten Strompfad durch das Gewebe (60) fließen kann,
**dadurch gekennzeichnet, dass**
die Hilfsneutralelektrode (50, 51, 52) eine bipolare Fasszange (50) umfasst, deren beiden als Pole dienenden Klemmflächen (51, 52) mit der Generatorschaltung (12) verbunden sind,
wobei der elektrochirurgische HF-Generator (10) eine Messschaltung (C1) zur Bestimmung des Widerstands zwischen den Klemmflächen (51, 52) der Fasszange (50) umfasst.

2. Elektrochirurgischer HF-Generator (10) nach Anspruch 1,
**dadurch gekennzeichnet, dass**
der elektrochirurgische HF-Generator (10) eine Messschaltung (C1) hat, die zumindest das Verhältnis der über die Neutralelektrode (40) und über die Hilfsneutralelektrode (50, 51, 52) fließenden HF-Ströme misst.

3. Elektrochirurgischer HF-Generator (10) nach einem der vorhergehenden Ansprüche,
**dadurch gekennzeichnet, dass**
der elektrochirurgische HF-Generator (10) eine Messschaltung (C1) hat, die den über die Neutralelektrode (40) und den über die Hilfsneutralelektrode (50, 51, 52) fließenden HF-Strom misst.

4. Elektrochirurgischer HF-Generator (10) nach Anspruch 1,
**dadurch gekennzeichnet, dass**
die Fasszange (50) selbsthaltend ist.

5. Elektrochirurgischer HF-Generator (10) nach einem der vorhergehenden Ansprüche,
**dadurch gekennzeichnet, dass**
der elektrochirurgische HF-Generator (10) einen Wahlschalter (S1, S2, S3, S6) zum Verbinden einer Klemmfläche (52) der bipolaren Fasszange (50) mit dem Aktivausgang (14) und zum Verbinden der anderen Klemmfläche (51) der bipolaren Fasszange mit dem Neutralausgang (13) umfasst.

6. Elektrochirurgischer HF-Generator (10) nach Anspruch 5,
**dadurch gekennzeichnet, dass**
der HF-Generator (10) einen Wahlschalter (S4, S5) zum Trennen der übrigen Elektroden (30, 40) von der Generatorschaltung (12), umfasst.

7. Elektrochirurgischer HF-Generator (10) nach einem der vorhergehenden Ansprüche,
**dadurch gekennzeichnet, dass**
der elektrochirurgische HF-Generator (10) einen Schalter (S2, S3; S7, S8) zum Trennen der Hilfsneutralelektrode (50, 51, 52) von der Generatorschaltung (12) umfasst.

8. Elektrochirurgischer HF-Generator (10) nach einem der vorhergehenden Ansprüche,
**dadurch gekennzeichnet, dass**
der elektrochirurgische HF-Generator (10) eine Messschaltung (C1) hat, die den elektrischen Widerstand zwischen der Neutralelektrode (40) und der Hilfsneutralelektrode (50, 51, 52) misst.

## Claims

1. Electrosurgical HF generator (10) for treating, in particular for cutting or coagulating, biological tissue (60) by means of an HF current which is generated by a generator circuit (12) and which flows via a first current path through the tissue (60) between an active electrode (30) and at least one neutral electrode (40) that contacts the tissue over a large area, with an active output (14) and a neutral output (13),
wherein the electrosurgical HF generator (10) has at least one auxiliary neutral electrode (50, 51, 52) which is connected in parallel to the neutral electrode (40) and which comprises at least one clamping surface (51, 52) of grasping forceps (50), such that at least some of the HF current can flow through the tissue (60) in a targeted manner via a second current path,
**characterized in that** the auxiliary neutral electrode (50, 51, 52) comprises bipolar grasping forceps (50), of which the two clamping surfaces (51, 52) serving as poles are connected to the generator circuit (12),
wherein the electrosurgical HF generator (10) comprises a measuring circuit (C1) for determining the resistance between the clamping surfaces (51, 52) of the grasping forceps (50).

2. Electrosurgical HF generator (10) according to Claim 1, **characterized in that** the electrosurgical HF generator (10) has a measuring circuit (C1) that measures at least the ratio of the HF currents flowing through the neutral electrode (40) and through the auxiliary neutral electrode (50, 51, 52).

3. Electrosurgical HF generator (10) according to one of the preceding claims, **characterized in that** the electrosurgical HF generator (10) has a measuring circuit (C1) that measures the HF current flowing through the neutral electrode (40) and the HF current flowing through the auxiliary neutral electrode (50, 51, 52).

4. Electrosurgical HF generator (10) according to Claim 1, **characterized in that** the grasping forceps (50) are self-locking.

5. Electrosurgical HF generator (10) according to one of the preceding claims, **characterized in that** the electrosurgical HF generator (10) comprises a selector switch (S1, S2, S3, S6) for connecting one clamping surface (52) of the bipolar grasping forceps (50) to the active output (14) and for connecting the other clamping surface (51) of the bipolar grasping forceps to the neutral output (13).

6. Electrosurgical HF generator (10) according to Claim 5, **characterized in that** the HF generator (10) comprises a selector switch (S4, S5) for separating the remaining electrodes (30, 40) from the generator circuit (12).

7. Electrosurgical HF generator (10) according to one of the preceding claims, **characterized in that** the electrosurgical HF generator (10) comprises a switch (S2, S3; S7, S8) for separating the auxiliary neutral electrode (50, 51, 52) from the generator circuit (12).

8. Electrosurgical HF generator (10) according to one of the preceding claims, **characterized in that** the electrosurgical HF generator (10) has a measuring circuit (C1) that measures the electrical resistance between the neutral electrode (40) and the auxiliary neutral electrode (50, 51, 52).

## Revendications

1. Générateur HF électrochirurgical (10) pour le traitement, en particulier pour la coupe ou la coagulation d'un tissu biologique (60) au moyen d'un courant HF à haute fréquence, généré par un circuit générateur (12), lequel circule entre une électrode active (30) et au moins une électrode neutre (40) entrant en contact avec le tissu sur une grande surface, sur un premier trajet de courant à travers le tissu (60), avec une sortie active (14) et une sortie neutre (13),
dans lequel le générateur HF électrochirurgical (10) possède au moins une électrode neutre auxiliaire (50, 51, 52) montée parallèlement à l'électrode neutre (40), laquelle comprend au moins une surface de serrage (51, 52) d'une pince de préhension (50) de sorte qu'au moins une partie du courant de HF puisse circuler de manière ciblée sur un deuxième trajet de courant à travers le tissu (60),
**caractérisé en ce que**
l'électrode neutre auxiliaire (50, 51, 52) comprend une pince de préhension bipolaire (50) dont les deux surfaces de serrage (51, 52) servant de pôles sont reliées au circuit générateur (12), dans lequel le générateur HF électrochirurgical (10) comprend un circuit de mesure (C1) pour la détermination de la résistance entre les surfaces de serrage (51, 52) de la pince de préhension (50).

2. Générateur HF électrochirurgical (10) selon la revendication 1,
**caractérisé en ce que**
le générateur HF électrochirurgical (10) possède un circuit de mesure (C1) qui mesure au moins le rapport des courants de HF circulant via l'électrode neutre (40) et via l'électrode neutre auxiliaire (50, 51, 52).

3. Générateur HF électrochirurgical (10) selon l'une des revendications précédentes,
**caractérisé en ce que**
le générateur HF électrochirurgical (10) possède un circuit de mesure (C1) qui mesure le courant circulant via l'électrode neutre (40) et le courant circulant via l'électrode neutre auxiliaire (50, 51, 52).

4. Générateur HF électrochirurgical (10) selon la revendication 1,
**caractérisé en ce que**
la pince de préhension (50) est auto-adhésive.

5. Générateur HF électrochirurgical (10) selon l'une des revendications précédentes,
**caractérisé en ce que**
le générateur HF électrochirurgical (10) comprend un commutateur sélecteur (S1, S2, S3, S6) pour relier une surface de serrage (52) de la pince de préhension bipolaire (50) à la sortie active (14) et pour relier l'autre surface de serrage (51) de la pince de préhension bipolaire à la sortie neutre (13).

6. Générateur HF électrochirurgical (10) selon la revendication 5,
**caractérisé en ce que**
le générateur HF (10) comprend un commutateur sélecteur (S4, S5) pour couper du circuit générateur (12) les autres électrodes (30, 40).

7. Générateur HF électrochirurgical (10) selon l'une des revendications précédentes,
**caractérisé en ce que**
le générateur HF électrochirurgical (10) comprend un interrupteur (S2, S3 ; S7, S8) pour couper du circuit générateur (12) l'électrode neutre auxiliaire (50, 51, 52).

8. Générateur HF électrochirurgical (10) selon l'une des revendications précédentes,
**caractérisé en ce que**
le générateur HF électrochirurgical (10) possède un circuit de mesure (C1) qui mesure la résistance électrique entre l'électrode neutre (40) et l'électrode neutre auxiliaire (50, 51, 52).
